# EUROPÄISCHE PATENTSCHRIFT

(11) **EP 3 912 606 B1**
(45) Veröffentlichungstag und Bekanntmachung des Hinweises auf die Patenterteilung: **04.10.2023**
(21) Anmeldenummer: 21175000.5
(22) Anmeldetag: 20.05.2021
(51) Int. Cl.: A61F 9/008, A61F 9/009

(54) **VERFAHREN ZUM BEREITSTELLEN VON STEUERDATEN FÜR EINEN AUGENCHIRURGISCHEN LASER EINER BEHANDLUNGSVORRICHTUNG**
METHOD FOR PROVIDING CONTROL DATA FOR AN OPHTHALMIC SURGICAL LASER OF A TREATMENT DEVICE
PROCÉDÉ DE FOURNITURE DES DONNÉES DE COMMANDE POUR UN LASER CHIRURGICAL OPHTALMIQUE D'UN DISPOSITIF DE TRAITEMENT

(30) Priorität: 22.05.2020 DE 102020113819
(43) Veröffentlichungstag der Anmeldung: 24.11.2021
(73) Patentinhaber: Schwind Eye-Tech-Solutions GmbH, 63801 Kleinostheim (DE)
(72) Erfinder: ARBA MOSQUERA, Samuel, 63739 Aschaffenburg (DE)
(74) Vertreter: Hofstetter, Schurack & Partner

(56) Entgegenhaltungen:
- DE-A1-102006 053 120
- DE-A1-102008 017 293
- US-A1- 2019 247 225

## Beschreibung

Die vorliegende Erfindung betrifft ein Verfahren zum Bereitstellen von Steuerdaten für einen augenchirurgischen Laser einer Behandlungsvorrichtung für die Abtrennung eines Lentikels. Die Erfindung betrifft außerdem eine Behandlungsvorrichtung mit mindestens einem augenchirurgischen Laser und mindestens einer Steuereinrichtung zum Durchführen des Verfahrens, ein Computerprogramm und ein computerlesbares Medium.

Behandlungsvorrichtungen und Verfahren zur Steuerung von ophthalmologischen Lasern zur Korrektur einer optischen Fehlsichtigkeit sind im Stand der Technik bekannt. Dabei können zum Beispiel ein gepulster Laser und eine Strahlfokussierungseinrichtung so ausgebildet sein, dass Laserstrahlpulse in einem innerhalb des organischen Materials gelegenen Fokus eine Photodisruption bewirken, um einen Lentikel aus der Hornhaut (Kornea) zur Korrektur der Fehlsichtigkeit abzutrennen. Bei der Behandlung mit einer Behandlungsvorrichtung zur Abtrennung eines Lentikels wird das Auge üblicherweise durch ein oder mehrere Kontaktelemente der Behandlungsvorrichtung fixiert. Das Kontaktelement ist hierbei ein starres Element, beispielsweise eine plankonkave Linse, die auf das Auge, insbesondere auf die Hornhaut (Kornea), aufgesetzt wird, damit das Auge bei der Behandlung nicht bewegt wird. Nachteilig bei einem solchen Kontaktelement ist es jedoch, dass eine Form der Hornhaut nicht exakt an das Kontaktelement angeglichen sein kann, wodurch sich die Hornhaut durch das Kontaktelement verformt. Hierdurch kann sich auch die Form des abzutrennenden Lentikels verändern, wodurch eine Behandlung fehlerbehaftet sein kann. Um dies auszugleichen ist es bekannt, Simulationen oder eine geometrische Änderung an einem Koordinatensystem durchzuführen. Diese Methoden sind jedoch sehr komplex und aufwändig durchzuführen.

Aus der DE 10 2008 017 293 A1 ist ein Verfahren zum Erzeugen von Steuerdaten für eine augenchirurgische Behandlungsvorrichtung bekannt, welche mittels einer Lasereinrichtung in der Augen-Hornhaut Gewebeschichten trennt, wobei im Betrieb der Lasereinrichtung ein eine Kontaktfläche aufweisendes Kontaktglas die Hornhaut in die Form der Kontaktfläche verformt, wozu die Kontaktfläche zuerst mit einem Kontaktflächenscheitel auf einen Hornhautscheitel gesetzt und dann zur Verformung der Hornhaut gegen diese gedrückt wird. In dem Verfahren werden die Steuerdaten für die Lasereinrichtung so erzeugt, dass sie Koordinaten von in der Hornhaut liegenden Zielpunkten für die Lasereinrichtung vorgeben, und bei der Erzeugung der Zielpunkt-Koordinaten wird die Verformung der Hornhaut berücksichtigt, welche während des Betriebs der Lasereinrichtung bedingt durch das Kontaktglas vorliegt.

Aus der US 2019/0247225 A1 ist eine Vorrichtung zur Erzeugung von Steuerdaten für eine Laservorrichtung zur chirurgischen Korrektur einer Fehlsichtigkeit bekannt. Das Verfahren umfasst ein Ändern einer Krümmung eines Augenhintergrundes durch Erzeugung eines Schnitts in der Hornhaut durch Einstrahlen von Laserstrahlung in die Hornhaut, wobei der Schnitt ein Volumen von Hornhautgewebe im Inneren der Hornhaut umschließt und isoliert, und ein Entfernen des umschlossenen Volumens von Hornhautgewebe aus dem Inneren der Hornhaut. Des Weiteren umfasst das Verfahren ein Herstellen des Schnitts, der das Volumen umschließt und isoliert, um einen vorderen und einen hinteren Teil zu umfassen, wobei der vordere Teil mit einem konstanten Abstand zu einer Hornhautvorderseite liegt, und wobei die Krümmung der Hornhaut des Auges durch unterschiedliches Entfernen des Volumens an zumindest zwei Radien r1 und r2 um die optische Achse geändert wird.

Aus der DE 10 2006 053 120 A1 ist eine Behandlungsvorrichtung zur operativen Fehlsichtigkeitskorrektur eines Auges eines Patienten bekannt, die eine von einer Steuereinrichtung gesteuerte Lasereinrichtung aufweist, welche durch Einstrahlen von Laserstrahlung Hornhaut-Gewebe trennt, wobei die Steuereinrichtung die Lasereinrichtung zur Fokussierung der Laserstrahlung in die Hornhaut auf in einem Muster in der Hornhaut liegende Zielpunkte ansteuert und das Muster so wählt, dass damit ein Volumen in der Hornhaut isoliert ist, dessen Entfernung aus der Hornhaut die gewünschte Fehlsichtigkeitskorrektur bewirkt.

Der Erfindung liegt die Aufgabe zugrunde, Steuerdaten zum Steuern eines augenchirurgischen Lasers bereitzustellen, bei denen auf einfache Art und Weise ein Ausgleich einer Deformation eines Lentikels bereitgestellt wird, die durch ein Kontaktelement erzeugt wird.

Diese Aufgabe wird durch das erfindungsgemäße Verfahren, die erfindungsgemäßen Vorrichtungen, das erfindungsgemäße Computerprogramm sowie das erfindungsgemäße computerlesbare Medium gelöst. Vorteilhafte Ausgestaltungen mit zweckmäßigen Weiterbildungen der Erfindung sind in den jeweiligen Unteransprüchen angegeben, wobei vorteilhafte Ausgestaltungen des Verfahrens als vorteilhafte Ausgestaltungen der Behandlungsvorrichtung, der Steuereinrichtung, des Computerprogramms und des

computerlesbaren Mediums und umgekehrt anzusehen sind.

Die Erfindung wird durch die Ansprüche definiert.

Ein erster Aspekt der Erfindung betrifft ein Verfahren zum Bereitstellen von Steuerdaten eines augenchirurgischen Lasers einer Behandlungsvorrichtung für die Abtrennung eines Lentikels, wobei das Verfahren die folgenden, durch eine Steuereinrichtung durchgeführten Schritte aufweist. Unter einer Steuereinrichtung wird dabei ein Gerät, eine Gerätekomponente oder eine Gerätegruppe verstanden, das/die zum Empfangen und Auswerten von Signalen eingerichtet ist, sowie zum Bereitstellen, zum Beispiel Erzeugen, von Steuerdaten. Die Steuereinrichtung kann zum Beispiel als Steuerchip, Computerprogramm, Computerprogrammprodukt oder Steuergerät ausgestaltet sein. Durch die Steuereinrichtung erfolgt ein Ermitteln einer Lentikelgeometrie des abzutrennenden Lentikels aus vorbestimmten Fehlsichtigkeitsdaten eines menschlichen oder tierischen Auges, wobei die Lentikelgeometrie mittels eines zu korrigierenden Brechkraftwerts und eines Lentikeldurchmessers definiert wird, und ein Ermitteln eines Korrekturwertes zum Ausgleich einer Deformation des Lentikels, die durch mindestens ein Kontaktelement der Behandlungsvorrichtung erzeugt wird, wobei der Korrekturwert mittels zumindest einer vorangegangenen Messung der Behandlungsvorrichtung bestimmt wird. Ferner erfolgt durch die Steuereinrichtung ein Ermitteln einer Deformationsgeometrie des Lentikels, wobei die Deformationsgeometrie mittels eines Deformationsbrechkraftwerts und eines Deformationsdurchmessers definiert wird, wobei der Deformationsbrechkraftwert in Abhängigkeit des zu korrigierenden Brechkraftwerts und des Korrekturwertes berechnet wird und der Deformationsdurchmesser in Abhängigkeit des Lentikeldurchmessers und des Korrekturwertes berechnet wird. Durch die Steuereinrichtung erfolgt schließlich ein Bereitstellen von Steuerdaten zum Steuern des augenchirurgischen Lasers, wobei die Steuerdaten die Deformationsgeometrie zur Abtrennung des Lenktikels verwenden.

Mit anderen Worten wird zunächst aus zuvor bestimmten Fehlsichtigkeitsdaten die Lentikelgeometrie des abzutrennenden Lentikels bestimmt. Die Fehlsichtigkeitsdaten können zum Beispiel eine Ametropie beschreiben, also ein Abweichen der Brechkraft eines Auges beziehungsweise einer Hornhaut vom Idealwert, zum Beispiel eine Kurzsichtigkeit, Weitsichtigkeit oder Stabsichtigkeit. Die Fehlsichtigkeitsdaten können zum Beispiel einen Wert einer Brechkraft beschreiben, also eine Angabe in Dioptrien, oder zum Beispiel eine Abweichung einer Hornhautverkrümmung von einem Normalwert. Die Fehlsichtigkeitsdaten können zum Beispiel durch Abrufen der Fehlsichtigkeitsdaten von einem Datenspeicher oder Datenserver erfolgen, oder die Fehlsichtigkeitsdaten können zum Beispiel aus einem Messgerät, das Abmessungen der Hornhaut vornimmt und/oder die Ametropie feststellt, empfangen werden. Anhand der festgestellten Fehlsichtigkeitsdaten ermittelt die Steuereinrichtung eine Lentikelgeometrie, welche die Abmessungen eines Lentikels und dessen Position in der Kornea beschreibt. Das heißt, die Lentikelgeometrie, also die gewünschte Form des Lentikels und dessen Position in der Hornhaut, wird mittels der Fehlsichtigkeitsdaten bestimmt, wobei insbesondere eine Lentikelhöhe mittels eines zu korrigierenden Brechkraftwerts und eines Lentikeldurchmessers, der auch optische Zone genannt wird, definiert wird. Unter einem Lentikel wird dabei ein Volumenkörper der Kornea verstanden, also zum Beispiel eine diskusförmige Scheibe, die durch anteriore und posteriore Grenzflächen definiert sein kann. Die Grenzflächen können beispielsweise einen bikonvexen, bikonkaven, konkavkonvexen, konvexkonkaven, planparallelen, plankonvexen oder plankonkaven Volumenkörper ausbilden.

Nach oder vor diesem Verfahrensschritt kann die Steuereinrichtung einen Korrekturwert zum Ausgleich einer Deformation des Lentikels ermitteln, wobei die Deformation des Lentikels durch das Kontaktelement der Behandlungsvorrichtung erzeugt wird. Der Korrekturwert wird hierbei aus zumindest einer vorangegangenen Messung durch die Behandlungsvorrichtung bestimmt. Das heißt, der Korrekturwert wird empirisch bestimmt, aus einer zuvor durchgeführten Messung mit der Behandlungsvorrichtung, beispielsweise aus einer klinischen oder präklinischen Messung. Im einfachsten Fall kann der Korrekturwert ein einzelner globaler Zahlenwert sein. Der Korrekturwert kann jedoch auch eine Funktion von gemessenen Parametern umfassen. Das heißt, dass für bestimmte Parameter ein eigener Korrekturwert vorgesehen sein kann. Beispielsweise kann je nach Art der Honrhautkrümmung, einer bekannten Krümmung des Kontaktelements oder eines patientenspezifischen Parameters, zum Beispiel des Patientenalters, ein eigener Korrekturwert vorgesehen sein.

Mittels des Korrekturwertes kann dann eine Deformationsgeometrie des Lentikels ermittelt werden, wobei hierfür aus dem zu korrigierenden Brechkraftwert und dem Korrekturwert ein Deformationsbrechkraftwert berechnet wird und aus dem Lentikeldurchmesser und dem Korrekturwert ein Deformationsdurchmesser. Aus dem Deformationsbrechkraftwert und dem Deformationsdurchmesser kann dann die Deformationsgeometrie bestimmt werden, die insbesondere die Form des Lentikels beschreibt, die zum Ausgleich der Deformation durch das mindestens eine Kontaktelement der Behandlungsvorrichtung verwendet werden soll. Schließlich können die Steuerdaten zum Steuern des augenchirurgischen Lasers bereitgestellt werden, die statt der Lentikelgeometrie die Deformationsgeometrie zur Abtrennung des Lentikels verwenden.

Des Weiteren ist vorgesehen, dass der Korrekturwert aus einer Abweichung des zu korrigierenden Brechkraftwerts und eines erreichten Brechkraftwerts ermittelt wird, wobei der erreichte Brechkraftwert nach einer Anwendung der Behandlungsvorrichtung ohne Verwendung der Deformationsgeometrie bestimmt wird. Mit anderen Worten kann die Abweichung bestimmt werden, die zwischen dem zu korrigierenden Brechkraftwert, das heißt dem geplanten Brechkraftwert, und dem erreichten Brechkraftwert vorliegt, wenn die Deformationsgeometrie nicht verwendet wird. Hieraus kann dann der Korrekturwert bestimmt werden. Beispielsweise kann geplant gewesen sein, dass der zu korrigierende Brechkraftwert acht Dioptrien aufweisen soll. Aufgrund der Deformation des Lentikels kann der erreichte Brechkraftwert jedoch nur sechs Dioptrien betragen haben. Das würde eine Abweichung von 25 Prozent ergeben, wobei der Korrekturwert vorzugsweise so ermittelt wird, dass diese 25 Prozent Abweichung ausgeglichen werden. Um diese Abweichung zwischen den Brechkraftwerten zu bestimmen, kann die Messung ohne Verwendung der Deformationsgeometrie vorzugsweise an künstlichen Hornhäuten und/oder an tierischen Hornhäuten und/oder an humanen Spenderhornhäuten durchgeführt werden. Hierdurch ergibt sich der Vorteil, dass der Korrekturwert mittels einer einfachen Messung bestimmt werden kann.

Durch diesen Aspekt der Erfindung ergibt sich der Vorteil, dass Deformationseffekte leicht ausgeglichen werden können und somit bessere Ergebnisse bei der Behandlung erzielt werden können. Des Weiteren kann durch die Anwendung eines aus vorangegangenen Messungen bestimmten Korrekturwertes auf eine aufwendige und komplexe Anwendung von Simulationen oder eine Änderung eines Koordinatensystems verzichtet werden.

Vorteilhaft ist es, wenn der Korrekturwert aus mehreren vorangegangenen Messungen des zu korrigierenden Brechkraftwerts und des erreichten Brechkraftwerts statistisch bestimmt wird, insbesondere mittels einer linearen Regression. Das hat den Vorteil, dass der Korrekturwert robuster gegenüber statistischen Abweichungen wird. Beispielsweise können mehrere Messungen durchgeführt werden, zum Beispiel für geplante Brechkraftwerte von beispielsweise sechs, sieben und acht Dioptrien, die jeweils mit dem erreichten Brechkraftwert verglichen werden. Des Weiteren können auch für einen zu korrigierenden Brechkraftwert mehrere Messungen durchgeführt werden, wobei diese dann mit den jeweils erreichten Brechkraftwerten statistisch verglichen werden. Hierzu können bekannte statistische Verfahren angewandt werden, insbesondere eine lineare Regression, deren Steigung die Abweichung zwischen den Brechkraftwerten darstellen kann.

Weiterhin vorteilhaft ist, wenn der erreichte Brechkraftwert zu mindestens zwei unterschiedlichen Zeitpunkten bestimmt wird, wobei der Korrekturwert aus einem Mittelwert der Abweichungen zu den mindestens zwei unterschiedlichen Zeitpunkten ermittelt wird. Insbesondere kann einer der Zeitpunkte kurz nach der Behandlung sein, beispielsweise einen Tag nach der Behandlung, und der andere Zeitpunkt kann später, beispielsweise nach einer Erholung des Auges von der Behandlung, zum Beispiel nach einer Woche oder einen Monat, sein. Der erreichte Brechkraftwert zu diesen zwei oder auch mehreren Zeitpunkten kann dann gemittelt werden, um die Abweichung vom geplanten Brechkraftwert und somit den Korrekturwert zu bestimmen. Das hat den Vorteil, dass auch eine Änderung des Brechkraftwerts in Abhängigkeit der Zeit berücksichtigt werden kann. Auch wird der Korrekturwert durch die Verwendung mehrerer Messungen statistisch robuster.

Vorzugsweise liegt der Korrekturwert in einem Bereich von 0,6 bis 1,4, insbesondere in einem Bereich von 0,7 bis 1,0. Das heißt, wenn zwischen dem zu korrigierenden Brechkraftwert und dem ermittelten Brechkraftwert eine Abweichung von 20 Prozent festgestellt wurde, kann der Korrekturwert bei 0,8 liegen. Insbesondere kann der Korrekturwert zwischen 0,7 und 1,0 liegen, da durch das Kontaktelement üblicherweise eine Unterkorrektur des Brechkraftwerts zu beobachten ist.

Gemäß einer weiteren vorteilhaften Ausgestaltungsform ist vorgesehen, dass eine Lentikelhöhe oder ein Lentikelvolumen bei der Berechnung der Deformationsgeometrie konstant gehalten werden und wobei der Deformationsbrechkraftwert mittels einer Multiplikation des zu korrigierenden Brechkraftwerts mit einem Kehrwert des Korrekturwerts berechnet wird. Mit anderen Worten wird der Deformationsbrechkraftwert berechnet, indem der zu korrigierende Brechkraftwert durch den Korrekturwert geteilt wird. Außerdem kann entweder die Lentikelhöhe oder das Lentikelvolumen für die Bestimmung der Deformationsgeometrie ausgehend von der Lentikelgeometrie beibehalten werden. Die Lentikelhöhe und das Lentikelvolumen sind hierbei Parameter der Lentikelgeometrie, die durch den Brechkraftwert und den Lentikeldurchmesser bestimmt werden können, wobei einer dieser Parameter bei der Berechnung der Deformationsgeometrie konstant gehalten werden kann. Vorzugsweise kann der Korrekturwert aus dem Verhältnis des erreichten Brechkraftwerts zu dem geplanten Brechkraftwert (zu korrigierenden Brechkraftwert) bestimmt werden, wobei hier der zu korrigierende Brechkraftwert mit dem Kehrwert des Korrekturwertes mittels einer Multiplikation berechnet wird. Für den Fachmann ist hierbei leicht zu erkennen, dass eine Änderung bei der Bestimmung des Korrekturwertes auch zu einer Änderung bei der Berechnung der Deformationsgeometrie führt. Beispielsweise kann bei einem umgekehrten Quotienten zur Bestimmung des Korrekturwertes der korrigierte Brechkraftwert direkt mittels einer Multiplikation mit dem Korrekturwert durchgeführt werden, da dies äquivalent ist.

Gemäß einer weiteren vorteilhaften Ausgestaltungsform wird der Deformationsdurchmesser mittels einer Multiplikation des Lentikeldurchmessers mit einem vom Korrekturwert abhängigen Parameter berechnet, wobei der Parameter mittels eines theoretischen Lentikelgeometriemodells, insbesondere mittels der Munnerlyn Formel, bestimmt wird. Mit anderen Worten kann zur Berechnung der Deformationsgeometrie der Deformationsdurchmesser aus dem Lentikeldurchmesser, das heißt dem geplanten Lentikeldurchmesser, berechnet werden, indem der Lentikeldurchmesser mit einem Parameter multipliziert wird, wobei der Parameter vom Korrekturwert abhängig ist. Mit dem vom Korrekturwert abhängigen Parameter ist gemeint, dass dieser eine mathematische Rechenoperation mit dem Korrekturwert umfasst, insbesondere eine Potenz des Korrekturwerts. Insbesondere kann der vom Korrekturwert abhängige Parameter aus einem Lentikelgeometriemodell bestimmt werden, vorzugsweise aus dem Lentikelgeometriemodell durch das die Lentikelgeometrie ermittelt wird, beispielsweise mittels der Munnerlyn Formel.

Weiterhin vorteilhaft ist, wenn bei einer konstant gehaltenen Lentikelhöhe der Deformationsdurchmesser mittels einer Multiplikation des Lentikeldurchmessers mit der Quadratwurzel des Korrekturwertes berechnet wird und bei einem konstant gehaltenen Lentikelvolumen der Deformationsdurchmesser mittels einer Multiplikation des Lentikeldurchmessers mit der vierten Wurzel des Korrekturwertes berechnet wird. Mit anderen Worten wird, falls die Lentikelhöhe konstant gehalten werden soll, der Deformationsdurchmesser berechnet, indem der Lentikeldurchmesser mit dem "Korrekturwert hoch ½" multipliziert wird. Falls das Lentikelvolumen konstant gehalten werden soll, wird der Deformationsdurchmesser vorzugsweise berechnet, indem der Lentikeldurchmesser mit dem "Korrekturwert hoch ¼" multipliziert wird. Das heißt, dass besonders bevorzugt zur Bestimmung der Deformationsgeometrie der Deformationsbrechkraftwert durch den Korrekturwert geteilt wird und gleichzeitig zur Berechnung des Deformationsdurchmessers der Lentikeldurchmesser mit der Quadratwurzel oder der vierten Wurzel des Korrekturwertes multipliziert wird, je nachdem, ob die Lentikelhöhe oder das Lentikelvolumen konstant gehalten werden sollen, wobei die Potenz des Korrekturwertes bei der Multiplikation des Lentikeldurchmessers vorzugsweise aus einer analytischen Berechnung des Lentikelgeometriemodells stammen, vorzugsweise aus einer Berechnung mittels der Munnerlyn Formel.

Ein zweiter Aspekt der vorliegenden Erfindung betrifft eine Steuereinrichtung, die dazu eingerichtet ist, eines der oben beschriebenen Verfahren durchzuführen. Es ergeben sich die oben aufgeführten Vorteile. Die Steuereinrichtung kann zum Beispiel als Steuerchip, Steuergerät oder Anwenderprogramm ("App") ausgestaltet sein. Die Steuereinrichtung kann vorzugsweise eine Prozessoreinrichtung aufweisen und/oder einen Datenspeicher. Unter einer Prozessoreinrichtung wird ein Gerät oder eine Gerätekomponente zur elektronischen Datenverarbeitung verstanden. Die Prozessoreinrichtung kann zum Beispiel mindestens einen Mikrocontroller und/oder mindestens einen Mikroprozessor aufweisen. Auf dem optionalen Datenspeicher kann vorzugsweise ein Programmcode zum Durchführen des erfindungsgemäßen Verfahrens abgelegt sein. Der Programmcode kann dann dazu ausgelegt sein, bei Ausführung durch die Prozessoreinrichtung die Steuereinrichtung dazu zu veranlassen, eine der oben beschriebenen Ausführungsformen eines oder beider erfindungsgemäßer Verfahren durchzuführen.

Ein dritter Aspekt der vorliegenden Erfindung betrifft eine Behandlungsvorrichtung mit mindestens einem augenchirurgischen Laser für die Abtrennung eines vordefinierten Hornhautvolumens mit vordefinierten Grenzflächen eines menschlichen oder tierischen Auges mittels Photodisruption, und mindestens einer Steuereinrichtung für den oder die Laser, die ausgebildet ist, die Schritte des Verfahrens gemäß dem ersten Aspekt der Erfindung auszuführen. Die erfindungsgemäße Behandlungsvorrichtung ermöglicht es, dass die bei der Verwendung üblicher ablativer Behandlungsvorrichtungen auftretenden Nachteile zuverlässig reduziert oder sogar vermieden werden.

In einer weiteren vorteilhaften Ausgestaltung der erfindungsgemäßen Behandlungsvorrichtung kann der Laser dazu geeignet sein, Laserpulse in einem Wellenlängenbereich zwischen 300 nm und 1400 nm, vorzugsweise zwischen 700 nm und 1200 nm, bei einer jeweiligen Pulsdauer zwischen 1 fs und 1 ns, vorzugsweise zwischen 10 fs und 10 ps, und einer Wiederholungsfrequenz größer 10 Kilohertz (KHz), vorzugsweise zwischen 100 KHz und 100 Megahertz (MHz), abzugeben. Ein solcher Femtosekundenlaser ist zur Herstellung von Volumenkörpern innerhalb der Kornea besonders gut geeignet. Die Verwendung von photodisruptiven Lasern bei dem erfindungsgemäßen Verfahren weist zudem den Vorteil auf, dass die Bestrahlung der Kornea nicht in einem Wellenlängenbereich unter 300 nm erfolgen muss. Dieser Bereich wird in der Lasertechnik unter dem Begriff "tiefes Ultraviolett" subsumiert. Dadurch wird vorteilhafterweise vermieden, dass durch diese sehr kurzwelligen und energiereichen Strahlen eine unbeabsichtigte Schädigung der Kornea erfolgt. Photodisruptive Laser der hier verwendeten Art bringen üblicherweise gepulste Laserstrahlung mit einer Pulsdauer zwischen 1 fs und 1 ns in das Korneagewebe ein. Dadurch kann die für den optischen Durchbruch notwendige Leistungsdichte des jeweiligen Laserpulses räumlich eng begrenzt werden, so dass eine hohe Schnittgenauigkeit bei der Erzeugung der Grenzflächen ermöglicht wird. Als Wellenlängenbereich kann insbesondere auch der Bereich zwischen 700 nm und 780 nm gewählt werden.

In weiteren vorteilhaften Ausgestaltungen der erfindungsgemäßen Behandlungsvorrichtung kann die Steuereinrichtung mindestens eine Speichereinrichtung zur zumindest temporären Speicherung von mindestens einem Steuerdatensatz aufweisen, wobei der oder die Steuerdatensätze Steuerdaten zur Positionierung und/oder zur Fokussierung einzelner Laserpulse in der Hornhaut/Kornea umfassen; und kann mindestens eine Strahleinrichtung zur Strahlführung und/oder Strahlformung und/oder Strahlablenkung und/oder Strahlfokussierung eines Laserstrahls des Lasers aufweisen. Die genannten Steuerdatensätze werden dabei üblicherweise anhand einer gemessenen Topografie und/oder Pachymetrie und/oder Morphologie der zu behandelnden Kornea und der Art der zu korrigierenden Fehlsichtigkeit, erzeugt.

Weitere Merkmale und deren Vorteile sind den Beschreibungen des ersten Erfindungsaspekts zu entnehmen, wobei vorteilhafte Ausgestaltungen jedes Erfindungsaspekts als vorteilhafte Ausgestaltungen des jeweils anderen Erfindungsaspekts anzusehen sind.

Ein vierter Aspekt der Erfindung betrifft ein Computerprogramm, umfassend Befehle, die bewirken, dass die Behandlungsvorrichtung gemäß dem dritten Erfindungsaspekt die Verfahrensschritte gemäß dem ersten Erfindungsaspekt und/oder die Verfahrensschritte gemäß dem zweiten Erfindungsaspekt ausführt.

Ein fünfter Aspekt der Erfindung betrifft ein computerlesbares Medium, auf dem das Computerprogramm gemäß dem vierten Erfindungsaspekt gespeichert ist. Weitere Merkmale und deren Vorteile sind den Beschreibungen des ersten bis vierten Erfindungsaspekts zu entnehmen, wobei vorteilhafte Ausgestaltungen jedes Erfindungsaspekts als vorteilhafte Ausgestaltungen des jeweils anderen Erfindungsaspekts anzusehen sind.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder von diesen abweichen. Dabei zeigt:
- Fig. 1: eine schematische Darstellung einer erfindungsgemäßen Behandlungsvorrichtung gemäß einer beispielhaften Ausführungsform;
- Fig. 2: ein beispielhaftes Streudiagramm von geplanten und erreichten Brech kraftwerten;
- Fig. 3: ein schematisches Verfahrensdiagramm gemäß einer beispielhaften Ausführungsform.

In den Figuren sind gleiche oder funktionsgleiche Elemente mit den gleichen Bezugszeichen versehen.

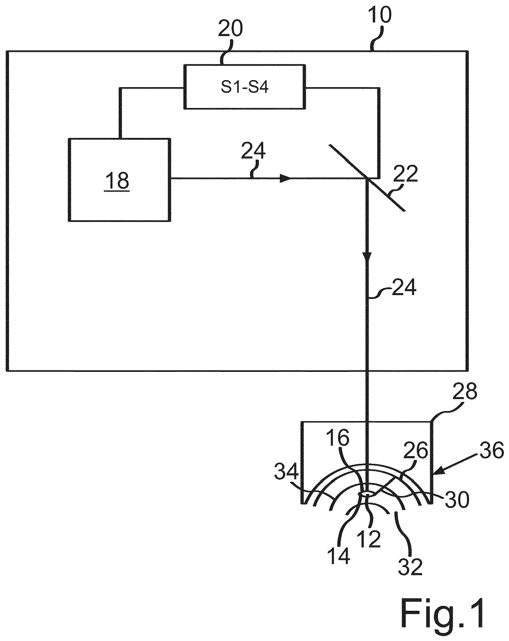

Die Figur 1 zeigt eine schematische Darstellung einer Behandlungsvorrichtung 10 mit einem augenchirurgischen Laser 18 für die Abtrennung eines durch eine Lentikelgeometrie vordefinierten Hornhautvolumens/Korneavolumens einer Hornhaut (Kornea) eines menschlichen oder tierischen Auges 36, also eines Lentikels 12, der auch als Volumenkörper bezeichnet werden kann, mit vordefinierten Grenzflächen 14, 16 mittels Photodisruption. Man erkennt, dass neben dem Laser 18 eine Steuereinrichtung 20 für den Laser 18 ausgebildet sein kann, sodass dieser gepulste Laserpulse beispielsweise in einem vordefinierten Muster in die Kornea des Auges 36 abgibt, wobei die ermittelten Grenzflächen 14, 16 des zu bildenden Lentikels 12 durch zum Beispiel ein vordefiniertes Muster mittels Photodisruption erzeugt werden können. Alternativ kann die Steuereinrichtung 20 eine in Bezug auf die Behandlungsvorrichtung 10 externe Steuereinrichtung 20 sein.

Die ermittelten Grenzflächen 14, 16 bilden in dem dargestellten Ausführungsbeispiel einen Lentikel 12 aus, wobei die Position des Lentikels 12 in diesem Ausführungsbeispiel derart gewählt ist, dass dieser zum Beispiel innerhalb einer Stroma 32 der Kornea liegen kann. Des Weiteren ist aus Figur 1 erkennbar, dass zwischen der Stroma 32 und einem Epithelium die so genannte Bowman-Membran 34 ausgebildet sein kann.

Des Weiteren zeigt die Figur 1, dass der durch den Laser 18 erzeugte Laserstrahl 24 mittels einer Strahleinrichtung 22, nämlich einer Strahlablenkungsvorrichtung, wie zum Beispiel einem Rotationscanner, in Richtung einer Oberfläche 26 der Hornhaut abgelenkt wird. Die Strahlablenkvorrichtung wird ebenfalls durch die Steuereinrichtung 20 gesteuert, um die ermittelten Grenzflächen 14, 16, vorzugsweise auch Inzisionen oder Schnitte 30, entlang von vorgegebenen Inzisionsverläufen zu erzeugen.

Bei dem dargestellten Laser 18 kann es sich vorzugsweise um einen photodisruptiven Laser handeln, der ausgebildet ist, Laserpulse in einem Wellenlängenbereich zwischen 300 nm und 1400 nm, vorzugsweise zwischen 700 nm und 1200 nm, bei einer jeweiligen Pulsdauer zwischen 1 fs und 1 ns, vorzugsweise zwischen 10 fs und 10 ps, und einer Wiederholungsfrequenz größer 10 KHz, vorzugsweise zwischen 100 KHz und 100 MHz, abzugeben. Die Steuereinrichtung 20 weist optional zudem eine Speichereinrichtung (nicht dargestellt) zur zumindest temporären Speicherung von mindestens einem Steuerdatensatz auf, wobei der oder die Steuerdatensätze Steuerdaten zur Positionierung und/oder zur Fokussierung einzelner Laserpulse in der Kornea umfassen. Die Positionsdaten und/oder Fokussierungsdaten der einzelnen Laserpulse, das heißt, die Lentikelgeometrie des abzutrennenden Lentikels 12, wird anhand vorbestimmter Fehlsichtigkeitsdaten erzeugt, insbesondere aus einer zuvor gemessenen Topografie und/oder Pachymetrie und/oder der Morphologie der Hornhaut oder der zu erzeugenden optischen Fehlsichtigkeitskorrektur beispielhaft innerhalb der Stroma 32 des Auges 36 erzeugt. Zum Feststellen der Fehlsichtigkeitsdaten, die zum Beispiel einen Wert in Dioptrien angeben können oder andere, geeignete Daten zum Beschreiben der Fehlsichtigkeit, kann die Steuereinrichtung 20 zum Beispiel die entsprechenden Daten von einem Datenserver empfangen, oder die Fehlsichtigkeitsdaten können als Dateneingabe festgestellt werden. Die Lentikelgeometrie des abzutrennenden Lentikels 12 zur Korrektur der Fehlsichtigkeit wird insbesondere mittels eines zu korrigierenden Brechkraftwerts, der auch als geplanter Brechkraftwert bezeichnet werden kann, und eines Lentikeldurchmessers, der auch als optische Zone bezeichnet wird, definiert.

Ferner kann ein Kontaktelement 28 bereitgestellt sein, das zu der Behandlungsvorrichtung 10 gehören kann. Alternativ kann das Kontaktelement 28 auch separat zu der Behandlungsvorrichtung 10 vorgesehen sein. Das Kontaktelement 28, das auch als Patienteninterface oder Fixiersystem bezeichnet werden kann, dient dazu, das Auge 36 für die Behandlung zu fixieren. Hierzu kann das Kontaktelement 28 eine plankonkave Linse aufweisen, die auf das Auge 36 zur Fixierung angepasst wird. Durch die Fixierung mittels des Kontaktelements 28 kann es jedoch dazu kommen, dass sich die Kornea verformt und somit die ermittelte Lentikelgeometrie zum Abtrennen des Lentikels 12 nicht mehr optimal auf die vorbestimmten Fehlsichtigkeitsdaten zutrifft. Daher kann es vorkommen, dass ein geplanter beziehungsweise zu korrigierender Brechkraftwert von einem erreichten Brechkraftwert nach der Behandlung mit der Behandlungsvorrichtung 10 abweicht.

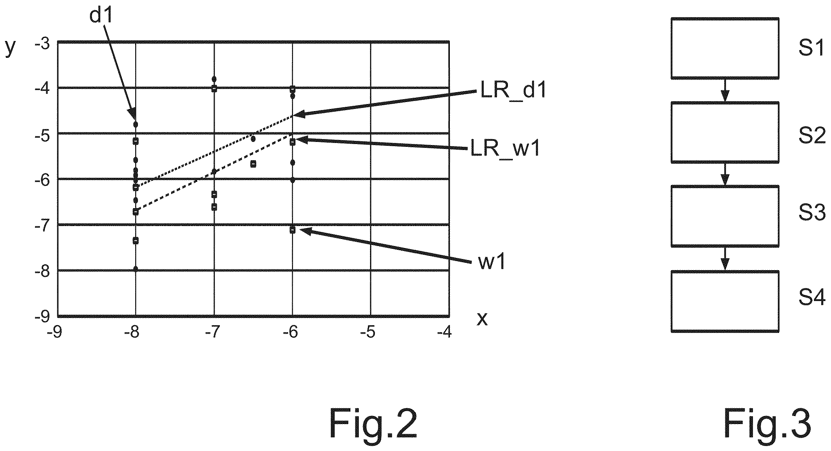

Dies ist in einem beispielhaften Streudiagramm der Fig. 2 veranschaulicht, wobei auf der Y-Achse der erreichte Brechkraftwert nach der Behandlung und auf der X-Achse der zu korrigierende Brechkraftwert, der mittels der vorbestimmten Fehlsichtigkeitsdaten geplant wurde, dargestellt ist. Das heißt, dass für jeden dargestellten Punkt der Wert auf der X-Achse geplant wurde und der Wert auf der Y-Achse erreicht wurde. Des Weiteren sind diese Abweichungen in der Fig. 2 für einen Tag nach der Behandlung (Kreise), zum Beispiel Datenpunkt d1, und für eine Woche nach der Behandlung (Quadrate), zum Beispiel Datenpunkt w1 aufgetragen.

Um diese durch das Kontaktelement 28 verursachte Deformation auszugleichen, kann vorgesehen sein, dass die Steuereinrichtung 20 die nachfolgend beschriebenen Verfahrensschritte S1 bis S4 durchführt, die beispielsweise in Fig. 3 als Verfahrensdiagramm dargestellt sind. In dem Schritt S1 wird, wie oben beschrieben, die Lentikelgeometrie des abzutrennenden Lentikels 12 aus den vorbestimmten Fehlsichtigkeitsdaten bestimmt, wobei die Lentikelgeometrie mittels des zu korrigierenden Brechkraftwerts und des Lentikeldurchmessers definiert wird. Anschließend kann in dem Schritt S2 ein Korrekturwert zum Ausgleich der Deformation durch das Kontaktelement 28 aus zumindest einer vorangegangenen Messung mit der Behandlungsvorrichtung 10 bestimmt werden. Hierzu kann beispielsweise das Streudiagramm aus Fig. 2 verwendet werden, wobei insbesondere eine lineare Regression LR_d1 der zu korrigierenden Brechkraftwerte und der erreichten Brechkraftwerte am ersten Tag nach der Behandlung statistisch bestimmt wird und vorzugsweise auch eine lineare Regression LR_w1 zu dem weiteren Zeitpunkt eine Woche nach der Behandlung gebildet wird. Beispielsweise kann eine Steigung der Regressionsgeraden LR_d1 einen Wert von 0,77 aufweisen, was einer 23 prozentigen Abweichung einen Tag nach der Behandlung entsprechen würde und die Regressionsgrade LR_w1 kann beispielsweise eine Steigung von 0,83 aufweisen, was einer 17 prozentigen Abweichung eine Woche nach der Behandlung entsprechen würde. Das heißt, dass in diesem Beispiel der Korrekturwert vorzugsweise zwischen 0,77 und 0,83 liegt, wobei der Korrekturwert vorzugsweise aus einem Mittelwert dieses Wertebereichs gebildet sein kann. Also in diesem Ausführungsbeispiel bei 0,8. Die zuvor gezeigten Zahlen sollen jedoch nur Beispiele des Korrekturwertes darstellen und der Korrekturwert kann beispielsweise je nach Behandlungsvorrichtung und dazugehörigem Kontaktelement unterschiedlich sein. Auch eine Art der Behandlung, das heißt in Abhängigkeit der Fehlsichtigkeitsdaten, kann den Korrekturwert verändern. Insbesondere kann der Korrekturwert in einem Bereich von 0,6 bis 1,4 liegen, vorzugsweise in einem Bereich von 0,7 bis 1,0.

Nach Ermittlung des Korrekturwerts kann durch die Steuereinrichtung in dem Schritt S3 eine Deformationsgeometrie des Lentikels ermittelt werden, wobei die Deformationsgeometrie mittels eines Deformationsbrechkraftwerts und eines Deformationsdurchmessers definiert wird. Der Deformationsbrechkraftwert kann in Abhängigkeit des zu korrigierenden Brechkraftwerts, das heißt dem zuvor geplanten Brechkraftwert aus der anfänglich geplanten Lentikelgeometrie, und des Korrekturwertes berechnet werden, indem der zu korrigierende Brechkraftwert durch den Korrekturwert geteilt wird. Mit anderen Worten wird der geplante Brechkraftwert durch den Korrekturwert, also gemäß dem Beispiel oben durch 0,8 geteilt, um den Deformationsbrechkraftwert zu erhalten. Des Weiteren kann im Schritt S3 gewählt werden, ob eine Lentikelhöhe oder ein Lentikelvolumen konstant gehalten wird, das heißt, ob die bei der Lentikelgeometrie ermittelte Lentikelhöhe für die Deformationsgeometrie übernommen wird oder das Lentikelvolumen.

Soll die Lentikelhöhe übernommen werden, kann ein Deformationsdurchmesser der Deformationsgeometrie in Abhängigkeit des Lentikeldurchmessers und des Korrekturwerts berechnet werden, indem der Lentikeldurchmesser mit einem vom Korrekturwert abhängigen Parameter berechnet wird, wobei der Parameter mittels eines theoretischen Lentikelgeometriemodells bestimmt wird. Beispielsweise kann das Lentikelgeometriemodell dasjenige Modell sein, mittels dem zuvor die Lentikelgeometrie bestimmt wurde. Vorzugsweise kann das Lentikelgeometriemodell die Munnerlyn Formel sein, wobei der vom Korrekturwert abhängige Parameter dabei die Quadratwurzel des Korrekturwertes ist. Das heißt, dass bei konstant gehaltener Lentikelhöhe der Deformationsdurchmesser berechnet wird, indem der Lentikeldurchmesser mit der Quadratwurzel des Korrekturwertes, also im Beispiel oben mit der Wurzel von 0,8 multipliziert wird. Soll das Lentikelvolumen konstant gehalten werden, wird ein anderer vom Korrekturwert abhängiger Parameter mittels des theoretischen Lentikelmodells berechnet, der im Falle der Munnerlyn Formel der vierten Wurzel des Korrekturwertes entspricht. Das heißt, dass der Deformationsdurchmesser bei konstant gehaltenem Lentikelvolumen mittels einer Multiplikation des Lentikeldurchmessers mit der vierten Wurzel des Korrekturwertes berechnet wird, also im Beispiel oben mit 0,8 hoch 0,25.

Die so bestimmte Deformationsgeometrie kann dann in einem Schritt S4 in Form von Steuerdaten zum Steuern des augenchirurgischen Lasers 18 von der Steuereinrichtung 20 bereitgestellt werden. Mittels der so bereitgestellten Steuerdaten, die die Deformationsgeometrie zur Abtrennung des Lentikels verwenden, kann auf einfache Art und Weise ein Ausgleich der Deformation durch das Kontaktelement 28 erreicht werden, wodurch eine Behandlung mit der Behandlungsvorrichtung 10 verbessert werden kann. Auch kann mittels des Verfahrens die Behandlung kontinuierlich verbessert werden, da der Korrekturwert iterativ angepasst werden kann. Beispielsweise kann der Korrekturwert zunächst mit 0,8 bestimmt werden. Nach weiteren Messungen unter Anwendung dieses Korrekturwerts kann dann beispielsweise immer noch eine Abweichung von 1,05 ermittelt werden, woraufhin der ursprüngliche Korrekturwert von 0,8 mit 1,05 multipliziert wird und somit einen iterativ angepassten Korrekturwert von 0,84 ergibt. Folglich kann der Korrekturwert auch iterativ für die Behandlungsvorrichtung verfeinert werden.

## Patentansprüche

1. Verfahren zum Bereitstellen von Steuerdaten eines augenchirurgischen Lasers (18) einer Behandlungsvorrichtung (10) für die Abtrennung eines Lentikels (12), wobei das Verfahren die folgenden, durch eine Steuereinrichtung (20) durchgeführten Schritte aufweist:
- Ermitteln (S1) einer Lentikelgeometrie des abzutrennenden Lentikels (12) aus vorbestimmten Fehlsichtigkeitsdaten eines menschlichen oder tierischen Auges (36), wobei die Lentikelgeometrie mittels eines zu korrigierenden Brechkraftwerts und eines Lentikeldurchmessers definiert wird;
- Ermitteln (S2) eines Korrekturwertes zum Ausgleich einer Deformation des Lentikels (12), die durch mindestens ein Kontaktelement (28) der Behandlungsvorrichtung (10) erzeugt wird, wobei der Korrekturwert mittels zumindest einer vorangegangenen Messung der Behandlungsvorrichtung (10) bestimmt wird;
- Ermitteln (S3) einer Deformationsgeometrie des Lentikels (12), wobei die Deformationsgeometrie mittels eines Deformationsbrechkraftwerts und eines Deformationsdurchmessers definiert wird, wobei der Deformationsbrechkraftwert in Abhängigkeit des zu korrigierenden Brechkraftwerts und des Korrekturwertes berechnet wird und der Deformationsdurchmesser in Abhängigkeit des Lentikeldurchmessers und des Korrekturwertes berechnet wird;
- Bereitstellen (S4) von Steuerdaten zum Steuern des augenchirurgischen Lasers (18), die die Deformationsgeometrie zur Abtrennung des Lentikels (12) verwenden; **gekennzeichnet dadurch, dass** der Korrekturwert aus einer Abweichung des zu korrigierenden Brechkraftwerts und eines erreichten Brechkraftwerts ermittelt wird, wobei der erreichte Brechkraftwert nach einer Anwendung der Behandlungsvorrichtung (10) ohne Verwendung der Deformationsgeometrie bestimmt wird.

2. Verfahren nach Anspruch 1
**dadurch gekennzeichnet, dass**
der Korrekturwert aus mehreren vorangegangenen Messungen des zu korrigierenden Brechkraftwerts und des erreichten Brechkraftwerts statistisch bestimmt wird, insbesondere mittels einer linearen Regression.

3. Verfahren nach einem der vorhergehenden Ansprüche
**dadurch gekennzeichnet, dass**
der erreichte Brechkraftwert zu mindestens zwei unterschiedlichen Zeitpunkten bestimmt wird, wobei der Korrekturwert aus einem Mittelwert der Abweichungen zu den mindestens zwei unterschiedlichen Zeitpunkten ermittelt wird.

4. Verfahren nach einem der vorhergehenden Ansprüche,
**dadurch gekennzeichnet, dass**
der Korrekturwert in einem Bereich von 0,6 bis 1,4 liegt, insbesondere in einem Bereich von 0,7 bis 1,0.

5. Verfahren nach einem der vorhergehenden Ansprüche,
**dadurch gekennzeichnet, dass**
eine Lentikelhöhe oder ein Lentikelvolumen bei der Berechnung der Deformationsgeometrie konstant gehalten werden und wobei der Deformationsbrechkraftwert mittels einer Multiplikation des zu korrigierenden Brechkraftwerts mit einem Kehrwert des Korrekturwertes berechnet wird.

6. Verfahren nach einem der vorhergehenden Ansprüche,
**dadurch gekennzeichnet, dass**
der Deformationsdurchmesser mittels einer Multiplikation des Lentikeldurchmessers mit einem vom Korrekturwert abhängigen Parameter berechnet wird, wobei der Parameter mittels eines theoretischen Lentikelgeometriemodells, insbesondere mittels der Munnerlyn Formel, bestimmt wird.

7. Verfahren nach Anspruch 5 und 6,
**dadurch gekennzeichnet, dass**
bei einer konstant gehaltenen Lentikelhöhe der Deformationsdurchmesser mittels einer Multiplikation des Lentikeldurchmessers mit der Quadratwurzel des Korrekturwertes berechnet wird und bei einem konstant gehaltenen Lentikelvolumen der Deformationsdurchmesser mittels einer Multiplikation des Lentikeldurchmessers mit der vierten Wurzel des Korrekturwertes berechnet wird.

8. Steuereinrichtung (20), die dazu ausgebildet ist, ein Verfahren nach einem der vorhergehenden Ansprüche durchzuführen.

9. Behandlungsvorrichtung (10) mit mindestens einem augenchirurgischen Laser (18) für die Abtrennung eines Lentikels (12) eines menschlichen oder tierischen Auges (36) mittels Photodisruption und mindestens einer Steuereinrichtung (20) nach Anspruch 8.

10. Behandlungsvorrichtung (10) nach Anspruch 9,
**dadurch gekennzeichnet, dass**
der Laser dazu ausgebildet ist, Laserpulse in einem Wellenlängenbereich zwischen 300 nm und 1400 nm, vorzugsweise zwischen 700 nm und 1200 nm, bei einer jeweiligen Pulsdauer zwischen 1 fs und 1 ns, vorzugsweise zwischen 10 fs und 10 ps, und einer Wiederholungsfrequenz größer 10 KHz, vorzugsweise zwischen 100 KHz und 100 MHz, abzugeben.

11. Behandlungsvorrichtung (10) nach einem der Ansprüche 9 oder 10,
**dadurch gekennzeichnet, dass** die Steuereinrichtung (20)
- mindestens eine Speichereinrichtung zur zumindest temporären Speicherung von mindestens einem Steuerdatensatz aufweist, wobei der oder die Steuerdatensätze Steuerdaten zur Positionierung und/oder Fokussierung einzelner Laserpulse in der Kornea umfassen; und
- mindestens eine Strahleinrichtung (22) zur Strahlführung und/oder Strahlformung und/oder Strahlablenkung und/oder Strahlfokussierung eines Laserstrahls (24) des Lasers umfasst.

12. Computerprogramm, umfassend Befehle, die bewirken, dass die Behandlungsvorrichtung (10) gemäß einem der Ansprüche 9 bis 11 ein Verfahren nach einem der Ansprüche 1 bis 7 ausführt.

13. Computerlesbares Medium, auf welchem das Computerprogramm nach Anspruch 12 gespeichert ist.

## Claims

1. A method for providing control data of an eye surgical laser (18) of a treatment apparatus (10) for the separation of a lenticule (12), wherein the method comprises the following steps performed by a control device (20):
- ascertaining (S1) a lenticule geometry of the lenticule (12) to be separated from predetermined visual disorder data of a human or animal eye (36), wherein the lenticule geometry is defined by means of a refractive power value to be corrected and a lenticule diameter;
- ascertaining (S2) a correction value for compensating for a deformation of the lenticule (12), which is generated by at least one contact element (28) of the treatment apparatus (10), wherein the correction value is determined by means of at least one preceding measurement of the treatment apparatus (10);
- ascertaining (S3) a deformation geometry of the lenticule (12), wherein the deformation geometry is defined by means of a deformation refractive power value and a deformation diameter, wherein the deformation refractive power value is calculated depending on the refractive power value to be corrected and the correction value and the deformation diameter is calculated depending on the lenticule diameter and the correction value;
- providing (S4) control data for controlling the eye surgical laser (18), which uses the deformation geometry for separating the lenticule (12);
**characterized in that**
the correction value is ascertained from a deviation of the refractive power value to be corrected and an achieved refractive power value, wherein the achieved refractive power value is determined after an application of the treatment apparatus (10) without use of the deformation geometry.

2. The method according to claim 1,
**characterized in that**
the correction value is statistically determined from multiple preceding measurements of the refractive power value to be corrected and the achieved refractive power value, in particular by means of a linear regression.

3. The method according to any one of the preceding claims,
**characterized in that**
the achieved refractive power value is determined at least at two different points of time, wherein the correction value is ascertained from an average value of the deviations at the at least two different points of time.

4. The method according to any one of the preceding claims,
**characterized in that**
the correction value is in a range from 0.6 to 1.4, in particular in a range from 0.7 to 1.0.

5. The method according to any one of the preceding claims,
**characterized in that**
a lenticule height or a lenticule volume is kept constant in the calculation of the deformation geometry, and wherein the deformation refractive power value is calculated by means of a multiplication of the refractive power value to be corrected by a reciprocal value of the correction value.

6. The method according to any one of the preceding claims,
**characterized in that**
the deformation diameter is calculated by means of a multiplication of the lenticule diameter by a parameter depending on the correction value, wherein the parameter is determined by means of a theoretical lenticule geometry model, in particular by means of the Munnerlyn formula.

7. The method according to claim 5 and 6,
**characterized in that**
the deformation diameter is calculated by means of a multiplication of the lenticule diameter by the square root of the correction value with a lenticule height kept constant and the deformation diameter is calculated by means of a multiplication of the lenticule diameter by the fourth root of the correction value with a lenticule volume kept constant.

8. A control device (20), which is formed to perform a method according to claim 1.

9. A treatment apparatus (10) with at least one eye surgical laser (18) for the separation of a lenticule (12) of a human or animal eye (36) by means of photodisruption and at least one control device (20) according to claim 8.

10. The treatment apparatus (10) according to claim 9,
**characterized in that**
the laser is formed to emit laser pulses in a wavelength range between 300 nm and 1400 nm, preferably between 700 nm and 1200 nm, at a respective pulse duration between 1 fs and 1 ns, preferably between 10 fs and 10 ps, and a repetition frequency of greater than 10 kHz, preferably between 100 kHz and 100 MHz.

11. The treatment apparatus (10) according to claim 9 or 10,
**characterized in that** the control device (20)
- comprises at least one storage device for at least temporary storage of at least one control dataset, wherein the control dataset or datasets include control data for positioning and/or focusing individual laser pulses in the cornea; and
- includes at least one beam device (22) for beam guidance and/or beam shaping and/or beam deflection and/or beam focusing of a laser beam (24) of the laser.

12. A computer program including instructions, which cause the treatment apparatus (10) according to any one of the claim 9 to 11 to execute a method according to any one of the claims 1 to 7.

13. A computer-readable medium, on which the computer program according to claim 12 is stored.

## Revendications

1. Procédé pour fournir des données de commande d'un laser chirurgical ophtalmique (18) d'un dispositif de traitement (10) pour la séparation d'un lenticule (12), le procédé comportant les étapes suivantes réalisées par un dispositif de commande (20) :
- déterminer (S1) une géométrie de lenticule du lenticule (12) à séparer à partir de données prédéterminées concernant un trouble visuel d'un oeil humain ou animal (36), la géométrie de lenticule étant définie au moyen d'une valeur de réfringence à corriger et d'un diamètre de lenticule ;
- déterminer (S2) une valeur de correction pour compenser une déformation du lenticule (12) qui est générée par au moins un élément de contact (28) du dispositif de traitement (10), la valeur de correction étant établie au moyen d'au moins une mesure précédente du dispositif de traitement (10) ;
- déterminer (S3) une géométrie de déformation du lenticule (12), la géométrie de déformation étant définie au moyen d'une valeur de réfringence avec déformation et d'un diamètre de déformation, la valeur de réfringence avec déformation étant calculée en fonction de la valeur de réfringence à corriger et de la valeur de correction, et le diamètre de déformation étant calculé en fonction du diamètre de lenticule et de la valeur de correction ;
- fournir (S4) des données de commande afin de commander le laser chirurgical ophtalmique (18), lesquelles données utilisent la géométrie de déformation pour séparer le lenticule (12) ;
**caractérisé en ce que**
la valeur de correction est déterminée à partir d'une déviation de la valeur de réfringence à corriger et d'une valeur de réfringence atteinte, la valeur de réfringence atteinte étant établie après une application du dispositif de traitement (10) sans utiliser la géométrie de déformation.

2. Procédé selon la revendication 1,
**caractérisé en ce que**
la valeur de correction est établie statistiquement à partir de plusieurs mesures précédentes de la valeur de réfringence à corriger et de la valeur de réfringence atteinte, en particulier au moyen d'une régression linéaire.

3. Procédé selon l'une des revendications précédentes,
**caractérisé en ce que**
la valeur de réfringence atteinte est établie à au moins deux instants différents, la valeur de correction étant déterminée à partir d'une valeur moyenne des déviations aux au moins deux instants différents.

4. Procédé selon l'une des revendications précédentes,
**caractérisé en ce que**
la valeur de correction est dans une plage de 0,6 à 1,4, en particulier dans une plage de 0,7 à 1,0.

5. Procédé selon l'une des revendications précédentes,
**caractérisé en ce que**
une hauteur de lenticule ou un volume de lenticule sont maintenus constants lors du calcul de la géométrie de déformation et dans lequel la valeur de réfringence avec déformation est calculée au moyen d'une multiplication de la valeur de réfringence à corriger par une valeur inverse de la valeur de correction.

6. Procédé selon l'une des revendications précédentes,
**caractérisé en ce que**
le diamètre de déformation est calculé au moyen d'une multiplication du diamètre de lenticule par un paramètre dépendant de la valeur de correction, le paramètre étant établi au moyen d'un modèle théorique de géométrie de lenticule, en particulier au moyen de la formule de Munnerlyn.

7. Procédé selon les revendications 5 et 6,
**caractérisé en ce que**
pour une hauteur de lenticule maintenue constante, le diamètre de déformation est calculé au moyen d'une multiplication du diamètre de lenticule par la racine carrée de la valeur de correction et, pour un volume de lenticule maintenu constant, le diamètre de déformation est calculé au moyen d'une multiplication du diamètre de lenticule par la racine quadratique de la valeur de correction.

8. Dispositif de commande (20) configuré pour mettre en oeuvre un procédé selon l'une des revendications précédentes.

9. Dispositif de traitement (10) comportant au moins un laser chirurgical ophtalmique (18) pour la séparation d'un lenticule (12) d'un oeil humain ou animal (36) au moyen d'une photodisruption, et au moins un dispositif de commande (20) selon la revendication 8.

10. Dispositif de traitement (10) selon la revendication 9,
**caractérisé en ce que**
le laser est conçu pour émettre des impulsions laser dans une gamme de longueurs d'onde entre 300 nm et 1 400 nm, de préférence entre 700 nm et 1 200 nm, à une durée d'impulsion respective entre 1 fs et 1 ns, de préférence entre 10 fs et 10 ps, et une fréquence de répétition supérieure à 10 kHz, de préférence entre 100 kHz et 100 MHz.

11. Dispositif de traitement (10) selon l'une des revendications 9 ou 10,
**caractérisé en ce que**
- le dispositif de commande (20) comporte au moins un dispositif de stockage pour le stockage au moins temporaire d'au moins un ensemble de données de commande, l'ensemble ou les ensembles de données de commande comprenant des données de commande pour le positionnement et/ou la focalisation d'impulsions laser individuelles dans la cornée ; et
- au moins un dispositif à faisceau (22) pour le guidage de faisceau et/ou la conformation de faisceau et/ou la déviation de faisceau et/ou la focalisation de faisceau d'un faisceau laser (24) du laser.

12. Programme informatique comprenant des instructions qui amènent le dispositif de traitement (10) selon l'une des revendication 9 à 11 à mettre en oeuvre un procédé selon l'une des revendications 1 à 7.

13. Support lisible par ordinateur sur lequel est stocké le programme informatique selon la revendication 12.
